# EUROPÄISCHE PATENTSCHRIFT

(11) **EP 1 397 375 B1**
(45) Veröffentlichungstag und Bekanntmachung des Hinweises auf die Patenterteilung: **28.04.2010**
(21) Anmeldenummer: 02780830.2
(22) Anmeldetag: 12.06.2002
(51) Int. Cl.: C07H 15/04, C07H 1/06

(54) **ALKYL-UND/ODER ALKENYLOLIGOGLYCOSID-ZUBEREITUNGEN MIT VERMINDERTEN MAGNESIUMSALZKONZENTRATIONEN**
ALKYL- AND/OR ALKENYLOLIGOGLYCOSIDE PREPARATIONS HAVING REDUCED MAGNESIUM SALT CONCENTRATIONS
PREPARATIONS ALKYL- ET/OU ALKENYLOLIGOGLYCOSIDE A CONCENTRATION REDUITE EN SEL DE MAGNESIUM

(30) Priorität: 21.06.2001 DE 10129484
(43) Veröffentlichungstag der Anmeldung: 17.03.2004
(73) Patentinhaber: Cognis IP Management GmbH, 40589 Düsseldorf (DE)
(72) Erfinder: ALBERS, Thomas, 45721 Haltern (DE); SCHMID, Karl, HEINZ, 40822 Mettmann (DE); ESKUCHEN, Rainer, 40764 Langenfeld (DE); KÖHLER, Michael, 40822 Mettmann (DE)
(86) Internationale Anmeldenummer: PCT/EP2002/006425
(87) Internationale Veröffentlichungsnummer: WO 2003/000710

(56) Entgegenhaltungen:
- WO-A-99/26957
- BIERMANN M ET AL: "ALKYLPOLYGLUCOSIDE - TECHNOLOGIE UND EIGENSCHAFTEN. ÖALKYLPOLYGLUCOSIDES - TECHNOLOGY AND PROPERTIES" STARCH STARKE, WILEY-VCH VERLAG, WEINHEIM, DE, Bd. 45, Nr. 8, 1. August 1993 (1993-08-01), Seiten 281-288, XP000383292 ISSN: 0038-9056 in der Anmeldung erwähnt

## Beschreibung

### Gebiet der Erfindung

Die Erfindung betrifft ein Verfahren zur Entfernung von Magnesiumsalzen aus Alkyl- und/oder Alkenyloligoglycosid-Zubereitungen.

### Stand der Technik

Bei der Herstellung von Alkyl- und/oder Alkenyloligoglycosiden durch Umsetzung von Fettalkoholen mit Glucose wird im Verlauf der Synthese Magnesiumoxid zugesetzt. Um zu gewährleisten, dass Alkyl- und/oder Alkenyloligoglycoside ohne Zusatz von Konservierungsmittel hohe Lagerstabilitäten aufweisen, müssen derartige Zubereitungen auf möglichst basische pH-Werte eingestellt werden. Insbesondere bei pH-Werten oberhalb von 11,3 kommt es zu unerwünschten Trübungen bzw. zu einem Niederschlag, der auf das Ausfallen des Magnesiumoxides zurückzuführen ist. Die Behandlung der Alkyl- und/oder Alkenyloligoglycosid-Zubereitungen mit Komplexierungsmitteln führt zwar zwischenzeitlich zu klaren Pasten, jedoch können die nach wie vor in der Lösung enthaltenen Magnesiumsalze bei der Weiterverarbeitung möglicherweise als schwerlösliche Salze freigesetzt werden.

Aus der WO 99/26957 A1 ist ein Verfahren zur Herstellung von APG-Zubereitungen bekannt, wobei Glucosen mit Fettalkoholen in Gegenwart eines sauren Katalysators umgesetzt werden, um das Rohprodukt anschließend mit einem Magnesiumoxid neutralisiert und anschließend mit Wasserstoffperoxid gebleicht wird. Die in den Beispielen offenbarten Zubereitungen enthalten ca. 300 ppm an Magnesiumsalz. Im Übersichtsartikel Alkylpolyglycoside-Technologie und Eigenschaften aus Starch/Stärke 45 (1993), 281-288 wird eine Übersicht über Alkyloligoglycoside und deren Herstellverfahren gegeben ohne dass hierbei detaillierte Angaben gemacht werden, wie man Magnesiumsalze aus den entsprechenden Zubereitungen entfernen kann.

Dementsprechend hat die Aufgabe der vorliegenden Erfindung darin bestanden, Alkyl- und/oder Alkenyloligoglycosid-Zubereitungen zur Verfügung zu stellen, die verminderte Konzentrationen an

Magnesiumsalzen enthalten, die ebenfalls bei basischen pH-Werten farbstabil sind und ggf. microbielle Stabiltät aufweisen und somit über den gesamten pH-Bereich keine Trübungen hervorrufen.

Weiterhin sollte ein einfaches und kostengünstiges Verfahren zur Herstellung dieser Zubereitungen gefunden werden.

### Beschreibung der Erfindung

Gegenstand der vorliegenden Erfindung ist ein Verfahren zum Entfernen von Magnesiumsalze aus Alkyl- und/oder Alkenyloligoglycosiden, bei dem man Alkyl- und/oder Alkenyloligoglycosid-Zubereitungen mit einem Aktivsubstanzgehalt von 10 bis 70 Gew.% - bezogen auf die Gesamtzusammensetzung - mit Säuren auf einen pH-Wert von 4 bis 9,5 einstellt und anschließend über eine mit einem Ionenaustauscherharz beladene Säule aufreinigt, mit der Maßgabe, dass man die Alkyl- und/oder Alkenyloligoglycosid-Zubereitungen bei einer Viskosität von 20 bis 6000 mPas (Brookfield, RVF-Viskosimeter, Spindel 5, 10 rpm) auf die Säule aufbringt.

Überraschenderweise wurde gefunden, dass man Alkyl- und/oder Alkenyloligoglycosid-Zubereitungen mit verminderter Magnesiumsalzkonzentration (< 10 ppm) erhalten kann, indem man Alkyl- und/oder Alkenyloligoglycosid-Zubereitungen einer bestimmten Viskosität und Konzentration zunächst mit Säuren auf einen bestimmten pH-Wert einstellt und anschliessend über eine mit einem Ionenaustauscherharz beladene Säule aufreinigt. Besonders vorteilhaft ist, dass diese aufgereinigten Zubereitungen ebenfalls bei basischen pH-Werten farbstabil sind und sogar bei pH-Werten oberhalb von 11,5 mikrobielle Stabilät zeigen. Dementsprechend rufen derartige Zubereitungen über den gesamten pH-Bereich keine unerwünschten Trübungen hervor. Die Herstellung dieser Alkyl- und/oder Alkenyloligoglycosid-Zubereitungen mit verminderten Magnesiumsalzkonzentrationen ist vergleichsweise einfach durchführbar und relativ kostengünstig.

### Alkyl- und/oder Alkenyloligoglykoside

Die erfindungsgemäßen Alkyl- und Alkenyloligoglykoside weisen die Formel **(I)** auf,

**R¹O-[G]ₚ (I)**

in der R¹ für einen Alkyl- und/oder Alkenylrest mit 4 bis 22 und vorzugsweise 8 bis 18 Kohlenstoff atomen, G für einen Zuckerrest mit 5 oder 6 und vorzugsweise 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993**)**, B.Salka in Cosm.Toil. 108, 89 (1993**)** sowie J.Kahre et al. in SÖFW-Journal Heft 8, 598 (1995**)** verwiesen.

Die Alkyl- und/oder Alkenyloligoglykoside können sich von Aldosen bzw. Ketosen mit 5 oder 6 Kohlen-stoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligo-glykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 22 und vorzugsweise 8 bis 11 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelenschen Oxosynthese erhalten werden. Bevorzugt sind Alkyloligo-glucoside der Kettenlänge C₈-C₁₀ (DP = 1 bis 3), die als Vorlauf bei der destillativen Auftrennung von technischem C₈-C₁₈-Kokosfeffalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner vorzugsweise ebenfalls von primären Alkoholen mit 12 bis 18 und insbesondere 12 bis 14 und 16 bis 18 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Vorzugsweise werden Alkyl- und/oder Alkenyloligoglycosid-Zubereitungen aufgereinigt, die eine Konzentration von 50 bis 2500, vorzugsweise 100 bis 1000 und insbesondere 400 bis 600 ppm Magnesium - bezogen auf die Gesamtzusammensetzung - enthalten. Unter Magnesiumsalzen werden im Sinne der Erfindung Magnesiumoxid/Magnesiumhydroxid, Magnesiumcarbonate, Magnesiumhalogenide und vorzugsweise Magnesiumoxid verstanden. Vorzugsweise werden im Sinne der Erfindung Alkyl- und/oder Alkenyloligoglycosid-Zubereitungen eingesetzt, die einen Aktivsubstanzgehalt von 10 bis 70, vorzugsweise 20 bis 67 und insbesondere 40 bis 65 Gew.-% besitzen.

### Ionenaustauscherharze (Harze)

Ionenaustauscher sind feste Stoffe oder flüssige Lösungen, welche fähig sind, positiv oder negativ geladene Ionen aus einer wässrigen Elektrolyt-Lösung unter Abgabe äquivalenter Mengen anderer Ionen aufzunehmen. Entsprechend der elektrischen Ladung der am Austausch beteiligten Ionen spricht man von Kationen- und Anionenaustauscher. Weiterhin können die Ionenaustauscher entsprechend ihrer Matrix und funktionellen Gruppen eingeteilt werden. Am häufigsten werden feste Körner und Partikel von Ionenaustauscher-Harzen, deren Matrix durch Kondensation (Phenol-Formaldehyd) oder durch Polymerisation (Copolymere aus Styrol und Divinylbenzol sowie Methacrylaten und Divinylbenzol) erhalten wurden, eingesetzt.

Im Sinne der vorliegenden Erfindung werden Ionenaustauscherharze eingesetzt, deren Matrix aus Styrol-Divinylbenzol-Copolymeren besteht und die als funktionelle Gruppen chelatbildende Gruppen, welche O-, N-, S- oder P-Atome beinhalten und damit für den Austausch von Magnesiumsalzionen geeignet sind, tragen.

Zu den chelatbildenden Gruppen zählen beispielsweise Sulfonsäure-, Carboxyl-, Aminomethylphosphonsäure- und Iminodiacetatgruppen. Vorzugsweise werden als funktionelle Gruppen Aminomethylphosphonsäure-, Iminodiacetat- und Sulfonsäuregruppen, wie beispielsweise Lewatit TP 260 (mit Aminomethylphosphonsäuregruppen) und Lewatit TP 207 und Lewatit TP 208 (mit Iminodiacetatgruppen) der Fa. Bayer oder Dowex Marathon C, Dowex Marathon C-10, Dowex Monosphere C 400, Dowex Marathon MSC (mit Sulfonsäuregruppen) der Fa. Dow oder Ambeelite 200 C, Amberlite 252, Amberlite IR120 (mit Sulfonsäuregruppen) der Fa. Rohm & Haas, verwendet. Insbesondere werden Lewatit TP 260 und besonders bevorzugt Lewatit TP 207 und Lewatit TP 208 eingesetzt.

### Verfahren

### Regeneration und Konditionierung des Austauscherharzes

Die Aufreinigung der Alkyl- und/oder Alkenyloligoglycosid-Zubereitungen wird mit einem regenerierten und anschliessend konditioniertem Ionenaustauscherharz durchgeführt. Hierzu wird eine Ionenaustauschersäule mit dem Ionenaustauscherharz (Harz) befüllt, mit 2 Füllvolumen 2 molarer Salzsäure regeneriert und anschließend mit 4 Füllvolumen Wasser gewaschen. Die Konditionierung des Harzes wird mit 1 bis 2 Füllvolumina 1 molarer Natronlauge vorzugsweise mit 2 Füllvolumen und anschliessend mit 3 bis 4 Füllvolumen Wasser gewaschen. Durch die Konditionierung wird beispielsweise bei Verwendung von Lewatit TP 207 die Mono- und/oder Di-Natrium-Form des Iminodiacetataustauscherharzes vorzugsweise die Di-Natrium-Form hergestellt. Die Fliessgeschwindigkeit bei der Regeneration und Konditionierung liegt vorzugsweise bei 1 bis 2 mm pro Sekunde.

Mit der Kapazität von ca. 1 kg Harz können bis zu ca. 100 kg Alkyl- und/oder Alkenyloligoglycosid-Zubereitung gereinigt werden.

### Behandlung der Alkyl- und/oder Alkenyloligoglycosid-Zubereitungen

Die Alkyl- und/oder Alkenyloligoglycosid-Zubereitungen werden auf eine Temperatur von 20 bis 90 °C, vorzugsweise von 30 bis 80°C und insbesondere von 40 bis 70°C vorgewärmt und mit einer Säure auf einen pH-Wert von 3 bis 9,5 und vorzugsweise 4 bis 9 und insbesondere 6 bis 8 eingestellt. Die Einstellung des pH-Wertes der Alkyl- und/oder Alkenyloligoglycosid-Zubereitungen erfiolgt vorzugsweise mit Mineralsäuren oder organischen Säuren. In einer besonderen Ausführungsform der Erfindung erfolgt die Einstellung des pH-Wertes mit einer 50 %igen Mineralsäure, wie beispielsweise Schwefelsäure, Salzsäure oder einer 50%igen organischen Säure, wie beispielsweise Citronensäure oder Weinsäure.

Die Viskosität der behandelten Alkyl- und/oder Alkenyloligoglycosid-Zubereitung sollte zwischen 20 und 6000, vorzugsweise zwischen 50 und 600 und insbesondere zwischen 100 und 400 mPas liegen.

Wird die Entfernung der Magnesiumsalze aus den Alkyl- und/oder Alkenyloligoglycosid-Zubereitungen im Durchlauf durch eine Ionenaustauschersäule durchgeführt und nicht durch Zugabe des Austauschers in die Zubereitung (bei gleichzeitigem Rühren und bei längerer Verweilzeit) mit anschliessender Entfernung des Ionenaustauschers nach erfolgter Entsalzung durch Filtration, so sollte die behandelte Zubereitung eine Viskosität zwischen 20 und 6000 mPas, bevorzugt von 50 bis 600 mPas und insbesondere von 100 bis 400 mPas (Brockfield, Spindel 5, 10 upm) aufweisen. Dadurch kann ein zu hoher Druckverlust in der Ionenaustauschersäule vermieden und eine Fliessgeschwindigkeit von bis zu 10 mm pro Sekunde - ggf. durch Anlegen eines leichten Überdruckes von 0,1 bis 2 bar wobei die Säule auf die Temperatur der vorgewärmten Alkyl- und/oder Alkenyloligoglycosid-Zubereitung durch äußere Beheizung erwärmt wird - gewährleistet werden. Sofern die Alkyl- und/oder Alkenyloligoglycosid-Zubereitungen höhere Viskositäten aufweisen wird zur Verminderung der Viskosität auf Temperaturen zwischen 20 bis 90°C, bevorzugt von 30 bis 80°C und insbesondere von 40 bis 70 °C erwärmt.

Die Zubereitung wird bei der oben angegebenen Viskosität auf die Ionenaustauschersäule mit dem regenerierten und konditionierten Austauscherharz (Harz) aufgetragen und der Ablauf aus der Säule auf den Magnesiumgehalt geprüft. Durch dieses Verfahren können Alkyl- und/oder Alkenyloligoglycosid-Zubereitungen erhalten werden, die eine Konzentration von < 10 und vorzugsweise < 7 und insbesondere < 1 ppm Magnesiumsalze - bezogen auf die Gesamtzusammensetzung - enthalten.

In einer besonderen Ausführungsform der Erfindung wird die behandelte Alkyl- und/oder Alkenyloligoglycosid-Zubereitung solange im Kreislauf durch die Ionenaustauschersäule gepumpt bis der Magnesiumgehalt < 10 und vorzugsweise < 7 und insbesondere < 1 ppm beträgt. Die Überprüfung des Magnesiumgehaltes wird mit den gängigen analytischen Methoden durchgeführt (z.B. mittels ICP-Chromatographie [Q-C 2085.0] oder mit Hilfe eines photometrischen Gehalttests -Küvettentest der Fa. Dr. Lange, LCK 326-).

In einer weiteren Verfahrensvariante ist ebenfalls denkbar das Ionenaustauscherharz in die neutralisierte Alkyl- und/oder Alkenyloligoglycosid-Zubereitung einzugeben, solange zu rühren bis der Magnesiumgehalt den geforderten Magnesiumgehalt unterschritten hat und anschließend zur Abtrennung des Ionenaustauscherharzes die Zubereitung durch einen 400µm-Beutelfilter zu filtrieren.

Im Anschluss können die aufgereinigten Alkyl- und/oder Alkenyloligoglycosid-Zubereitungen beispielsweise durch Behandlung mit einem Anionenaustauscher auf einen basischen pH-Wert eingestellt werden. Durch diesen Neutralisationsschritt können die bei der Neutralisation eingebrachten Anionen aus der Zubereitung entfernt und zum anderen der ursprüngliche basische pH-Wert, beispielsweise oberhalb von 11,3, wieder hergestellt werden.

Dementsprechend werden in einer bevorzugten Ausführungsform der Erfindung Alkyl- und/oder Alkenyloligoglycosid-Zubereitungen mit einer Magnesiumsalzkonzentration < 10, vorzugsweise < 7 und insbesondere < 1 ppm - bezogen auf die Gesamtzusammensetzung - erhalten, indem man Alkyl- und/oder Alkenyloligoglycosid-Zubereitungen mit einem Aktivsubstanzgehalt von 20 bis 67 und insbesondere 40 bis 65 Gew.-% bei Temperaturen von 30 bis 80°C und insbesondere von 40 bis 70°C mit Mineralsäuren oder organischen Säuren auf einen pH-Wert von 4 bis 9 insbesondere 6 bis 8 und anschliessend bei einer Viskosität von 50 bis 600 und vorzugsweise 100 bis 400 mPas (Brookfield, RVF-Viskosimeter, Spindel 5, 10 rpm) auf eine Ionenaustauschersäule, die mit einem regenerierten und anschliessend konditioniertem Ionenaustauscherharz beladen wurde, aufträgt und aufreinigt.

### Gewerbliche Anwendbarkeit

Die erfindungsgemäß hergestellten Alkyl- und/oder Alkenyloligoglycosid-Zubereitungen mit Magnesiumsalzkonzentrationen < 10 ppm können in allen den Fachmann bekannten oberflächenaktiven Zubereitungen, wie vorzugsweise in Wasch- und Spülmittel, Haushaltsreiniger sowie kosmetische und/oder pharmazeutische Zubereitungen und insbesondere in kosmetischen Zubereitungen für die Haar- und Körperpflege sowie in Reinigungsmitteln, wie beispielsweise Glasreiniger, eingesetzt werden. Diese oberflächenaktiven Zubereitungen können als weitere Hilfs- und Zusatzstoffe Perlglanzwachse, Konsistenzgeber, Verdickungsmittel, Überfettungsmittel, Stabilisatoren, Siliconverbindungen, Fette, Wachse, Lecithine, Phospholipide, Antioxidantien, Deodorantien, Antitranspirantien, Antischuppenmittel, Quellmittel, Tyrosininhibitoren, Hydrotrope, Solubilisatoren, Konservierungsmittel, Parfümöle, Farbstoffe, weitere Tenside und dergleichen enthalten. Als kosmetische und/oder pharmazeutische Zubereitungen kommen beispielsweise Mund- und Zahnpflegemittel, Haarshampoos, Haarlotionen, Schaumbäder, Duschbäder, Cremes, Gele, Lotionen, alkoholische und wässrig/alkoholische Lösungen und Emulsionen in Betracht.

### Beispiele

### 1. Regeneration und Konditionierung des Austauscherharzes

Eine Ionenaustauschersäule mit 10I Volumen wurde mit 2 kg Lewatit TP 207 (Fa. Bayer) befüllt, mit 5,4I 2 molarer Salzsäure regeneriert und anschließend mit 10,8I Wasser gewaschen. Die Konditionierung wurde mit 2,7 I1 molaren Natronlauge durchgeführt und mit 10,8 I Wasser gewaschen. Die Fliessgeschwindigkeit bei der Regeneration und Konditionierung lag bei 1,4 mm pro Sekunde.

### 2. Behandlung von Glucopon 215 CSUP (C_{8/10}-Alkyl- und/oder Alkenyloligoglycosid)

50 kg Glucopon 215 CSUP (63 bis 65 Gew.-% Aktivsubstanzgehalt) mit einem Gehalt an Magnesiumsalzen von 750 ppm wurde auf 70°C vorgewärmt und mit einer 30%igen Schwefelsäure (ca. 1600 ml) auf einen pH-Wert von ca. 6,7 eingestellt. Diese Zubereitung durchströmte die Ionenaustauschersäule mit einer Fliessgeschwindigkeit von bis zu 10 mm pro Sekunde ggf. durch Anlegen eines geringen Überdruck von 0,1 bis 1 bar bei einer Temperatur von 70°C durch äußere Beheizung der Säule. Im Ablauf aus der lonenaustauschersäule wurde der Magnesiumgehalt des behandelten Glucopon 215 CSUP mittels ICP (Atomfluoreszenzspektroskopie) kontrolliert. Die Konzentration an Magnesiumsalzen in der Zubereitung lag unter 10 ppm.

### 3. Lagerversuche

Glucopon 215 CSUP (63 bis 65 Gew.-% Aktivsubstanzgehalt) mit einem Gehalt an Magnesiumsalzen von 750 ppm und einem pH-Wert von 11,5 wurde bei 60 °C in einer 1-Liter-Glasflasche während 4 Wochen gelagert. Nach dieser Zeit zeigte sich in der Glasflasche ein Niederschlag an Magnesiumhydroxid von ca. 10 % des Gesamtvolumens.

Das nach Beispiel 2 gereingte Glucopon 215 CSUP wurde mit 50%iger Natronlauge auf einen pH-Wert von 11,5 eingestellt und bei 60°C in einer 1-Liter-Glasflasche während 4 Wochen gelagert. Nach dieser Zeit zeigte das Produkt keinen Niederschlag, sondern war wie zu Beginn des Lagertests klar-blank.

## Patentansprüche

1. Verfahren zum Entfernen von Magnesiumsalzen aus Alkyl- und/oder Alkenyloligoglycosiden, bei dem man Alkyl- und/oder Alkenyloligoglycosid-Zubereitungen mit einem Aktivsubstanzgehalt von 10 bis 70 Gew.-% - bezogen auf die Gesamtzusammensetzung - mit Säuren auf einen pH-Wert von 4 bis 9,5 einstellt und anschließend über eine mit einem Ionenaustauscherharz beladene Säule aufreinigt, mit der Maßgabe, dass man die Alkyl- und/oder Alkenyloligoglycosid-Zubereitungen bei einer Viskosität von 20 bis 6000 mPas (Brookfield, RVF-Viskosimeter, Spindel 5, 10 rpm) auf die Säule aufbringt.

2. Verfahren nach Anspruch 1, **dadurch gekennzeichnet, dass** man Alkyl- und Alkenyloligoglykoside der Formel (I) einsetzt,
R¹O-[G]ₚ (I)
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Verfahren nach den Ansprüchen 1 und/oder 2, **dadurch gekennzeichnet, dass** man Alkyl- und Alkenyloligoglykoside der Formel **(I)** einsetzt, in der R¹ für einen Alkyl- und/oder Alkenylrest mit 8 bis 18 Kohlenstoffatomen, G für einen Zuckerrest mit 5 oder 6 Kohlenstoffatomen und p für Zahlen von 1 bis 10 steht.

4. Verfahren nach mindestens einem der Ansprüche 1 bis 3, **dadurch gekennzeichnet, dass** man die Alkyl- und/oder Alkenyloligoglycosid-Zubereitungen auf einen pH-Wert von 5 bis 9 einstellt.

5. Verfahren nach mindestens einem der Ansprüche 1 bis 4, **dadurch gekennzeichnet, dass** man die Alkyl- und/oder Alkenyloligoglycosid-Zubereitungen bei einer Viskosität von 50 bis 600 mPas (Brookfield, RVF-Viskosimeter, Spindel 5, 10 rpm) und bei einer Temperatur von 30 bis 80°C auf die Säule aufbringt.

6. Verfahren nach mindestens einem der Ansprüche 1 bis 5, **dadurch gekennzeichnet, dass** man Mineralsäuren oder organische Säuren zur Neutralisation der Alkyl- und/oder Alkenyloligoglycosid-Zubereitungen einsetzt.

7. Verfahren nach mindestens einem der Ansprüche 1 bis 6, **dadurch gekennzeichnet, dass** man Alkyl- und/oder Alkenyloligoglycosid-Zubereitungen mit einem Aktivsubstanzgehalt von 20 bis 67 Gew.-% - bezogen auf die Gesamtzusammensetzung - einsetzt.

8. Verfahren nach mindestens einem der Ansprüche 1 bis 7, **dadurch gekennzeichnet, dass** man Ionenaustauscherharze mit einer Matrix aus Styrol-Divinylbenzol-Copolymeren einsetzt, die als funktionelle Gruppe Sulfonsäure-, Carboxyl-, Aminomethylphosphonsäure- oder Iminodiacetatgruppen tragen.

9. Verfahren nach mindestens einem der Ansprüche 1 bis 8, **dadurch gekennzeichnet, dass** man die Aufreinigung der Alkyl- und/oder Alkenyloligoglycosid-Zubereitungen mit einem regenerierten und anschließend konditionierten Ionenaustauscherharz durchführt.

## Claims

1. A process for removing magnesium salts from alkyl and/or alkenyl oligoglycosides in which alkyl and/or alkenyl oligoglycoside preparations with an active substance content of 10 to 70% by weight, based on the composition as a whole, are adjusted with acids to a pH of 4 to 9.5 and are then purified over a column charged with an ion exchange resin, with the proviso that the alkyl and/or alkenyl oligoglycoside preparations are applied to the column with a viscosity of 20 to 6,000 mPas (Brookfield, RVF viscosimeter, spindle 5, 10 r.p.m.).

2. The process as claimed in claim 1, **characterized in that** alkyl and alkenyl oligoglycosides of the formula (I):
R¹O-[G]ₚ (I)
in which R¹ is an alkyl and/or alkenyl group containing 4 to 22 carbon atoms, G is a sugar residue containing 5 or 6 carbon atoms and p is numbers from 1 to 10, are used.

3. The process as claimed in claims 1 and/or 2,
**characterized in that** alkyl and alkenyl oligoglycosides of the formula (I) in which R¹ is an alkyl and/or alkenyl group containing 8 to 18 carbon atoms, G is a sugar residue containing 5 or 6 carbon atoms and p is numbers from 1 to 10 are used.

4. The process as claimed in at least one of claims 1 to 3, **characterized in that** the alkyl and/or alkenyl oligoglycoside preparations are adjusted to a pH of 5 to 9.

5. The process as claimed in at least one of claims 1 to 4, **characterized in that** the alkyl and/or alkenyl oligoglycoside preparations are applied to the column with a viscosity of 50 to 600 mPas (Brookfield, RVF viscosimeter, spindle 5, 10 r.p.m.) and at a temperature of 30 to 80°C.

6. The process as claimed in at least one of claims 1 to 5, **characterized in that** mineral acids or organic acids are used for neutralization of the alkyl and/or alkenyl oligoglycoside preparations.

7. The process as claimed in at least one of claims 1 to 6, **characterized in that** alkyl and/or alkenyl oligoglycoside preparations with an active substance content of 20 to 67% by weight, based on the composition as a whole, are used.

8. The process as claimed in at least one of claims 1 to 7, **characterized in that** ion exchange resins with a matrix of styrene/divinylbenzene copolymers carrying sulfonic acid, carboxyl, aminomethyl phosphonic acid or iminodiacetate groups as the functional group are used.

9. The process as claimed in at least one of claims 1 to 8, **characterized in that** the purification of the alkyl and/or alkenyl oligoglycoside preparations is carried out with a regenerated and subsequently conditioned ion exchange resin.

## Revendications

1. Procédé pour l'élimination de sels de magnésium à partir d'alkyloligoglycosides et/ou d'alcényloligoglycosides, dans lequel on ajuste à un pH de 4 à 9,5, à l'aide d'acides, des préparations d'alkyl- et/ou alcényloligoglycosides ayant une teneur en substance active de 10 à 70 % en poids - par rapport à la composition totale - et ensuite on purifie sur une colonne chargée d'une résine échangeuse d'ions, étant entendu qu'on injecte dans la colonne les préparations d'alkyl- et/ou alcényloligoglycosides à une viscosité de 20 à 6 000 mPa.s (Brookfield, viscosimètre RVF, broche 5, 10 tours/min).

2. Procédé selon la revendication 1, **caractérisé en ce qu'**on utilise des alkyl- et/ou alcényloligoglycosides de formule (I)
R¹O-[G]ₚ (I)
dans laquelle R¹ représente un radical alkyle et/ou alcényle ayant de 4 à 22 atomes de carbone, G représente un radical glucidique ayant 5 ou 6 atomes de carbone et p représente des nombres valant de 1 à 10.

3. Procédé selon la revendication 1 et/ou la revendication 2, **caractérisé en ce qu'**on utilise des alkyl- et/ou alcényloligoglycosides de formule (I), dans laquelle R¹ représente un radical alkyle et/ou alcényle ayant de 8 à 18 atomes de carbone, G représente un radical glucidique ayant 5 ou 6 atomes de carbone et p représente des nombres valant de 1 à 10.

4. Procédé selon au moins l'une des revendications 1 à 3, **caractérisé en ce qu'**on ajuste les préparations d'alkyl- et/ou alcényloligoglycosides à un pH de 5 à 9.

5. Procédé selon au moins l'une des revendications 1 à 4, **caractérisé en ce qu'**on injecte dans la colonne les préparations d'alkyl- et/ou alcényloligoglycosides à une viscosité de 50 à 600 mPa.s (Brookfield, viscosimètre RVF, broche 5, 10 tours/min) et à une température de 30 à 80°C.

6. Procédé selon au moins l'une des revendications 1 à 5, **caractérisé en ce que** pour la neutralisation des préparations d'alkyl- et/ou alcényloligoglycosides on utilise des acides minéraux ou des acides organiques.

7. Procédé selon au moins l'une des revendications 1 à 6, **caractérisé en ce qu'**on utilise des préparations d'alkyl- et/ou alcényloligoglycosides ayant une teneur en substance active de 20 à 67 % en poids - par rapport à la composition totale.

8. Procédé selon au moins l'une des revendications 1 à 7, **caractérisé en ce qu'**on utilise des résines échangeuses d'ions ayant une matrice à base de copolymères de styrène-divinylbenzène, qui portent en tant que groupe fonctionnel des groupes sulfo, carboxy, aminométhylphosphono ou iminodiacétate.

9. Procédé selon au moins l'une des revendications 1 à 8, **caractérisé en ce qu'**on effectue la purification des préparations d'alkyl- et/ou alcényloligoglycosides à l'aide d'une résine échangeuse d'ions régénérée et ensuite conditionnée.
